# EUROPEAN PATENT SPECIFICATION

(11) **EP 3 107 587 B1**
(45) Date of publication and mention of the grant of the patent: **27.03.2019**
(21) Application number: 15711859.7
(22) Date of filing: 20.02.2015
(51) Int. Cl.: A61L 27/18, A61L 27/52, A61Q 19/08, A61K 9/00

(54) **DERMOCOSMETIC FILLER AND USES THEREOF FOR AESTHETIC PURPOSES**
DERMOKOSMETISCHE FÜLLMASSE UND IHRE VERWENDUNG FÜR ÄSTHETISCHE ZWECKE
MATIÈRE DE REMPLISSAGE DERMOCOSMÉTIQUE ET SON UTILISATION À DES FINS ESTHÉTIQUES

(30) Priority: 20.02.2014 IT MI20140248
(43) Date of publication of application: 28.12.2016
(73) Proprietor: Mastelli S.r.l., 18038 Sanremo (IM) (IT)
(72) Inventor: CATTARINI MASTELLI, Laura, 18018 Taggia (IT); CATTARINI MASTELLI, Giulia, 16122 Genova (IT); CATTARINI MASTELLI, Silvia, 18038 Sanremo (IT)
(74) Representative: Coppo, Alessandro
(86) International application number: PCT/IB2015/051302
(87) International publication number: WO 2015/125117

(56) References cited:
- EP-A1- 0 346 189
- EP-A2- 2 407 147
- US-A1- 2010 316 683
- COSTA, DIANA ET AL: "Some novel aspects of DNA physical and chemical gels", Arkat , 5 April 2006 (2006-04-05), pages 161-172, XP002731459, DOI: 10.3998/ARK.5550190.0007.411 Retrieved from the Internet: URL:http://www.arkat-usa.org/get-file/2305 5/ [retrieved on 2014-10-22]
- TOPUZ, FUAT ET AL: "Rheological Behavior of Responsive DNA Hydrogels", MACROMOLECULES, vol. 41, no. 22, 2008, pages 8847-8854, XP002731460, (WASHINGTON, DC, UNITED STATES) ISSN: 0024-9297, DOI: 10.1021/MA801414P cited in the application

## Description

### FIELD OF THE INVENTION

The scope of the invention is defined by the claims. Any references in the description to methods of treatment refer to the compounds, pharmaceutical compositions and medicaments of the present invention for use in a method for treatment of the human (or animal) body by therapy or surgery.

The present invention relates to a dermal filler and to intradermal uses thereof for aesthetic medicine purposes.

In particular, the present invention relates to an injectable dermal filler which finds main application in the aesthetic field for reducing the depth of the wrinkles, depressions or the like with the simultaneous and gradual release of free nucleotides or polynucleotides which play a trophic and anti-aging function on the skin dermis.

### PRIOR ART

Skin aging is a chronic phenomenon that shows with the appearance of wrinkles, furrows, atrophy, relaxation and loss of skin elasticity, phenomena that become progressively more evident with age.

Skin aging is caused by a reduction in the number and the functional ability of fibroblasts and by a reduction of their metabolic synthetic ability which is also the base of the senile dermo-epidermal dystrophy. In senescent skin there occurs a thinning of the epidermis and a loss of elasticity that is expressed with the appearance of wrinkles and furrows. In aesthetic medicine, fillers are used to treat these signs of aging and to fill skin wrinkles or increase volumes.

Fillers are generally polymeric products injectable subcutaneously in the vicinity of wrinkles, skin furrows and in general in areas of the body where there is a sagging skin. Once injected, they mainly carry out a mechanical action of filling the tissues, causing an action of stretching and relaxation of the skin which reduces the depth of the epidermal furrows.

The fillers available on the market can be divided into two main categories, those based on biodegradable substances and those non-biodegradable. Non-biodegradable fillers are called permanent fillers because they are not absorbable and are generally encapsulated in fibrous tissues and are not subject to hydrolysis or phagocytosis. While exerting a prolonged action, however, permanent fillers have a higher risk of developing allergic and rejection reactions by the human body.

Typical examples of permanent fillers include liquid silicones, silicone particles in suspension, particles of acrylic hydrogel crosslinked with hyaluronic acid, calcium hydroxyapatite, polymethylmethacrylate microspheres dispersed in collagen and formulations of polyacrylamide gel.

Biodegradable fillers are called temporary because their effect wears off over time and their application needs to be renewed to maintain unchanged the filling effect. Biodegradable fillers are generally divided into two types, the intermediate duration ones and the long duration ones. The intermediate duration ones are absorbable after a few months of implantation and are made of materials subject to degradation by enzymatic hydrolysis, such as hyaluronic acid, and therefore they gradually lose the initial ability to fill and relax the tissues. Typically these polymers are biodegradable materials present in nature, with high biocompatibility and affinity with water.

However, these polymeric matrices, such as hyaluronic acid, are rapidly hydrolysed once implanted in a tissue, and the results obtained, although appreciable from an aesthetic point of view, do not last long.

In order to remedy these problems, the biodegradable polymer matrix have been modified so as to obtain polymers in crosslinked form with longer duration of action.

For example, currently, hyaluronic acids are available on the market which are treated with crosslinking agents so as to form covalent bonds which make the resulting polymer matrix less subject to enzymatic hydrolysis and enzymatic degradation, once injected into the tissues.

Because these crosslinked polymer matrices are less soluble and less prone to degradation, they behave as inert substances that play a mere tissue filling function, without making a contribution to improve the conditions of the tissues that have led to the formation or accelerated the formation of the wrinkle.

Some long duration of action fillers are also known which are made in synthetic polymer matrices. While having the advantage of being little biodegradable and thus having a long duration of action, these fillers are not free from drawbacks as they can cause allergic reactions or skin redness.

US2010316683 discloses subcutaneously or intradermally injectable hydrogel comprising crosslinked hyaluronic acid and free hyaluronic acid or salts thereof. At present, therefore, there is the need to have new fillers which have a prolonged filling duration and which, once implanted, have low risk of rejection phenomena or allergic reactions.

One of the objects of the invention therefore is to provide a filler that combines a medium to long term filling action with a biological treatment action aimed to recover the skin or tissue eutrophism in the body area where it is applied.

A further object is to provide a highly biocompatible filler that, when used, is almost free of side effects.

A further object of the invention is to provide an aesthetic or cosmetic treatment method that eliminates or substantially reduces the imperfections related to skin aging, the realization of which is almost devoid of the typical side effects of cosmetic surgery techniques and techniques for correction of imperfections.

### SUMMARY OF THE INVENTION

According to some aspects of the invention, the inventors have found that by injecting chemically treated polynucleotides subcutaneously or intradermally, wherein the nucleic acid polymer chain moiety is at least partially crosslinked with a biocompatible crosslinking agent, in particular covalently crosslinked DNA chains, the filling properties typical of medium-long duration fillers are combined with the cell eutrophic and regenerative characteristics of polynucleotides or DNA. Such nucleotides or DNA stimulate the fibroblasts, causing both a numerical increase and an increase in the secretory activity. This effect is an important advantage of this filler that adds the eutrophic and anti-aging function of the dermis and skin to the mechanical function of filling wrinkles or furrows.

According to some aspects of the present invention, the inventors have discovered that by chemically crosslinking nucleic acid polymer chain moiety with a biocompatible crosslinking agent, a cross-linked polynucleotide matrix with reduced biodegradability is obtained which finds application in the cosmetic field as filling material or polymer also provided with a cell restoring and regenerative activity.

According to a first aspect thereof, a filler is provided, based on polynucleotides wherein the nucleic acid polymeric chain is at least partially cross-linked with a biocompatible crosslinking agent. According to some embodiments, the crosslinked polynucleotides or DNA is obtained by crosslinking DNA or polynucleotides having a molecular weight distribution from 0.5 to 3000 kDalton.

According to some embodiments, the nucleotide polymeric chain is at least partially purified in particular to eliminate its genetic informational ability.

In some embodiments, the polynucleotide is polydeoxyribonucleotide (PDRN) wherein the nucleotide unit substantially contains deoxyribose as sugar.

Typically, in the dermal filler, repeated units of DNA nucleotide are crosslinked with a biocompatible crosslinking agent.

In some embodiments, the nucleic acid polymer chain moiety is in the form of gel. According to some embodiments, the dermal filler is in injectable form, in particular an injectable gel.

According to some aspects thereof, the disclosure relates to a composition containing the filler described above.

The composition of the disclosure is used in aesthetics according to any one of the embodiments defined in claims 1-9 or it finds use in the medical field, typically in aesthetic medicine, according to any one of the embodiments of claims 10-15.

The composition, when applied to the area of interest, combines the mechanical function of a dermal filling with a cell regeneration activity and a delay in the cell aging process due to the sustained release of polynucleotides to the tissues at the implantation area. Moreover, the crosslinking of the polydeoxyribonucleotide, DNA increases the persistence time of the composition, filler at the injection, implantation site.

For example, the filler composition can have a filling effect lasting from 4 to 12 months, from 5 to 10 months, from 6 to 8 months.

Typically, the composition of the disclosure is a composition in a form suitable for application by injection or by intradermal or subcutaneous implantation.

According to a first aspect thereof, the present invention provides a non-therapeutic method for treating skin imperfections or for filling skin furrows comprising the injection into the dermis or in the subcutaneous tissue of an effective amount of an injectable composition based on DNA or a nucleic acid polymer chain moiety at least partially chemically crosslinked with a biocompatible crosslinking agent.

According to certain embodiments, the non-therapeutic method of the invention is suitable to stretch, reduce, mitigate wrinkles, facial furrows or to reshape, fill the volumes of areas of the face or the body of an individual or to treat or prevent facial aging.

According to a second aspect, a therapeutic treatment method is provided that comprises the injection into the dermis or in the subcutaneous tissue of an effective amount of an injectable composition based on polynucleotides comprising a nucleic acid polymer chain moiety at least partially crosslinked with a biocompatible crosslinking agent.

According to certain aspects, the therapeutic treatment method can be applied in cosmetic surgery, plastic surgery or orthopedics.

### DETAILED DESCRIPTION OF THE INVENTION

The present invention in certain aspects thereof originates from having found new indications of use for polynucleotides (PN) or DNA covalently crosslinked by means of a biocompatible crosslinking reagent.

In particular, the invention originates from having found that the subcutaneous or intradermal graft of a composition or gel based on DNA stabilized by chemical crosslinking combines mechanical and filling properties with biological characteristics of cell regeneration that improve and prolong the aesthetic effect following the filling and stretching of the soft tissues.

In particular, the aesthetic effect obtained is the outcome of the extension of time of the mechanical filling action resulting from the crosslinking of the polynucleotides (PN), DNA present in the composition combined with the biological action exerted on the dermal cells by PN or DNA that are progressively released by the composition.

According to certain aspects, the present disclosure relates to the therapeutic and non-therapeutic uses of a composition of the type described.

According to a first aspect thereof, the present invention therefore relates to the cosmetic or aesthetic use of an injectable composition based on DNA or a nucleic acid polymer chain moiety at least partially crosslinked with a biocompatible crosslinking agent as dermal filler and cell regenerative or to increase the volume of a soft tissue.

Typically, for this use, the subcutaneous or intradermal injection of a cosmetically effective amount of the filler composition is provided.

In some embodiments, the polynucleotides or crosslinked DNA contains or is obtained by crosslinking polynucleotides that have a molecular weight distribution in the range from 0.5 to 3000 kDalton, from 30 to 2000 kDalton, from 600 to 1600 kDalton, from 50 to 1600 kDalton. For example, the polynucleotides before crosslinking with the curing agent have a numeric average molecular weight of about 350 kDalton.

Within the scope of the invention, the term "polynucleotide" means a filament or polymeric chain based on nucleotides, in particular constituting genetic material, typically DNA.

Suitable nucleotides are the monomers constituting the nucleic acids RNA and DNA, in particular the latter. Each nucleotide contains a purine or pyrimidine nitrogen base, a sugar with five carbon atoms, typically pentose, and at least one phosphate group. The sugar may be ribose or deoxyribose.

The purine nitrogen bases include adenine, guanine, xanthine, hypoxanthine, the pyrimidine ones include thymine cytosine, uracil.

Each nucleotide units can contain from one to four phosphate residues.

In some embodiments, polynucleotides comprise monomeric units of DNA, typically based on polydeoxyribonucleotides (PDNR).

In some embodiments, the crosslinked DNA can be from 1000 to 10,000, from 1500 to 5000 base pairs in length.

According to certain embodiments, the polynucleotides of the filler can be obtained by extraction, by means of conventional technologies, from natural sources of natural, plant or animal origin, such as algae, plankton, crustaceans or fish.

In some embodiments, the polynucleotides which are the fillers of the disclosure are obtained by depolymerisation of moieties of greater length DNA, optionally purified.

In some embodiments, the polynucleotides are moieties of filaments or DNA polymer chains obtained by shredding DNA filament or chains to reduce the molecular weight and partial purification to eliminate the ability to carry genetic information.

According to some embodiments, DNA based polynucleotides are purified by removing from 1 to 5%, 2 to 2.5% by weight of the total content of the original purine bases.

According to some embodiments, the polynucleotides are sterilized, in particular by heating up to a temperature from 110 to 130°C, for example at 121 °C for a period of time ranging from 15 to 20 minutes.

The polynucleotides of the dermal filler are at least partially cross-linked with a biocompatible crosslinking agent.

Suitable crosslinking agents include crosslinking agents based on dialdehydes and disulfides, such as divinylsulfones, crosslinking agents, PEG, diglycidyl ethers, bis-carbodiimides, bis-epoxides and mixtures thereof.

In certain embodiments, the composition contains DNA chemically cross-linked by means of biocompatible crosslinking agents.

Typically, the crosslinking reaction occurs between the amino groups on the nucleotide bases of DNA with a biocompatible crosslinking agent. In certain embodiments, the crosslinking of DNA leads to the formation of chemical DNA hydrogels having typical rheological behaviors as described for example by Fuat Topuz et al. "Rheological behavior of responsive DNA hydrogels", Macromolecules Washington DC USA, 41 (22), 8847-8854.

Suitable crosslinking agents to chemically crosslink DNA or polynucleotides include biocompatible bifunctional and multifunctional crosslinking agents. Suitable crosslinking agents include polyethylene glycol diglycil ether, polyethylene glycol epoxide, divinyl sulfone (DVS), pentaerythritol tetraglycidyl ether (PETGE), 1,4-butanediol diglycidyl ether (BDDE), 1,2-bis(2,3-epoxypropoxy)ethylene (EDGE), 1,2,7,8-diepoxyoctane (DEO), phenylene-bis-(ethyl)-carbodiimide, 1,6-hexane-ethylene bis-(ethylcarbodiimide), adipic dihydrazide (ADH), bis(sulfosuccinimidyl)suberate (BS), hexamethylenediamine (HMDA), 1-(2,3-epoxypropyl)-2,3-epoxycyclohexane, BTDE or mixtures thereof.

Typically, suitable polynucleotides have a crosslinking degree suitable to improve one of the skin flaws described herein.

According to some embodiments, the polynucleotides have a crosslinking degree comprised from 0.1 to 80%, from 1 to 40% 5 to 20% in relation to the molecular weight of the polynucleotide chain. Typically, the crosslinking degree can be expressed as the ratio of the weight percentage of the crosslinking agent in relation to the polynucleotides.

According to certain aspects of the invention, the use of a filler according to any one of the embodiments described above is provided for the production of an injectable composition to increase the volume of a human body tissue.

According to another aspect thereof, the present disclosure provides a composition comprising
- a dermal filler based on polynucleotides or a nucleic acid polymer chain moiety wherein the nucleic acid polymer chain is crosslinked,
- a physiologically acceptable carrier.

Typically, the composition is a composition in a form suitable for the application by subcutaneous or intradermal injection or by implantation in a human tissue.

According to some embodiments, the composition contains from 0.01 to 80%, from 0.5 to 60%, from 1 to 50%, or from 10 to 40% by weight of the polynucleotide or DNA or moiety thereof according to any embodiment described above.

Typically, the physiologically acceptable carrier of the composition is an excipient, carrier or diluent suitable for intradermal or intracutaneous administration. A suitable carrier is a liquid for parenteral administration, particularly subcutaneous or intradermal.

Suitable carriers in liquid form include water, preferably ultrapure or for pharmaceutical use, isotonic saline solution (0.9%), aqueous solutions with phosphate buffer (PBS), Ringer's solutions, or aqueous solutions containing one or more of carbonate, citrate, acetate, glycine and mixtures thereof.

According to some embodiments, the composition contains one or more pH regulating agents and/or buffer systems of the conventional type in the techniques of preparation of injectable pharmaceutical solutions.

For example, phosphate or tartrate buffers may be used to adjust the pH to physiologically compatible values.

Typically, the composition in injectable form is a sterile and pyrogen-free saline.

In some embodiments, the composition has a pH in the range from 4.5 to 8, from 6 to 7.6.

According to some embodiments, the composition is in the form of gel or hydrogel suitable for subcutaneous or intradermal injection.

In some embodiments, the filler composition is in the form of gel particles.

According to some embodiments, the filler composition forms a monophasic gel or hydrogel.

In some embodiments, the gel or hydrogel particles have a particle size equal to or less than 700 µm. For example, from 50 to 700 µm, or from 100 to 400 µm, or from 200 to 300 µm, measured as the mass median diameter by laser diffraction technique or by microscopic analysis.

According to other embodiments, the monophasic gel is almost free from gel particles.

According to some embodiments, the composition is in the form of suspension, in particular water-based.

According to some embodiments, the composition further comprises one or more physiologically acceptable excipients such as antioxidants, diluents, solvents, preservatives, bactericidal agents, stabilizers, emulsifiers, surfactants, buffers, humectants, dyes, or other excipients commonly used in cosmetic/pharmaceutical preparation techniques.

The composition may further contain one or more antioxidants such as vitamin C, sodium bisulfite, glutathione, coenzyme Q and mixtures thereof.

In some embodiments, the composition comprises further cosmetically active substances which can be administered by injection.

According to certain embodiments, the filler or filler composition is applied the by subcutaneous or intradermal injection.

In accordance with an aspect of the invention, a cosmetic or aesthetic treatment method is provided that comprises application of a cosmetically acceptable amount of a composition based on polynucleotides comprising a nucleic acid polymer chain moiety at least partially crosslinked with a biocompatible crosslinking agent.

The cosmetic or aesthetic treatment method of the invention may be applied in the treatment of any area of the body, such as the face, the lips, the area around the eyes, breasts, buttocks, and in particular to improve the aesthetic quality of a anatomical feature.

According to certain embodiments, the composition of the invention is suitable for use for aesthetic purposes, in particular to increase the volume of areas of the body, for example the lips, around the eyes, or in the treatment, prevention, distension of wrinkles, skin folds or corrugations, particularly those located on the face.

In some embodiments, the cosmetic treatment method involves the intradermal or subcutaneous injection of a composition according to any one of the embodiments previously described for filling wrinkles, facial corrugations, skin depressions or scars.

According to a second aspect, the invention relates to a therapeutic method of treatment comprising the injection of a therapeutically effective amount of an injectable composition based on polynucleotides comprising a nucleic acid polymer chain moiety at least partially crosslinked with a biocompatible crosslinking agent.

In certain embodiments, the composition of the invention may contain a local anesthetic, such as lidocaine, tetracaine, mepivacaine, procaine, benzocaine, and mixtures thereof, analgesics such as non-steroidal anti-inflammatory drugs, antimicrobials such as antibiotics for local use and mixtures thereof.

In some embodiments, the active principles contained in the composition of the present invention can be combined or mixed as active principles in intimate admixture with a suitable carrier and/or excipient according to pharmaceutical techniques.

According to some aspects of the present invention, the filler or the filler composition of the invention can be applied in the medical field, for example in surgery, in particular plastic surgery, dentistry, urology, orthopedics or ophthalmology.

According to some embodiments of this aspect of the invention, the filler or filler composition of the invention is for the treatment or for use in the treatment of a medical condition including anatomical malformations, in particular with an impact on the aesthetic appearance of the person, or in the urinary incontinence, in the forms of tissue adhesions in connective particulars, in vesicoureteral reflux, in gastroesophageal reflux disease or lipoatrophy.

In some embodiments, the filler or the composition of the invention are applied in reconstructive surgery, such as in maxillofacial or aesthetic surgery, for example in plastic surgery, for example in blepharoplasty or rhinoplasty.

In accordance with other aspects of the present invention, the filler or the filler composition can be applied in cosmetic and/or aesthetic field.

In some embodiments, the filler or filler composition is used with mixtures of non-crosslinked polynucleotides or with mixtures of crosslinked polynucleotides and crosslinked and/or not crosslinked hyaluronic acid.

In some embodiments, the filler or filler composition is used with mixtures of crosslinked polynucleotides or with crosslinked hyaluronic acid or with the following 4 components: crosslinked polynucleotides + non-crosslinked polynucleotides + crosslinked hyaluronic acid + non-crosslinked hyaluronic acid. Typically, mixtures of the previously described filler can also be used for cutanous infiltrations.

In accordance with an aspect of the invention, a method for the treatment of an area of the body of an individual for aesthetic purposes is provided comprising the application or administration to an individual of a filler or of a composition comprising a filler according to the disclosure.

According to a further aspect, the disclosure provides a process for the preparation of the filler or crosslinked polynucleotide according to the disclosure including preparing a solution of polynucleotides and adding a biocompatible crosslinking reagent, for example, BDDE, thereto.

Typically, the solvent medium used is water, preferably in pure form and/or a mixture of water and alcohol, such as ethyl alcohol.

Alternatively, in order to obtain the crosslinked PN of the invention, it is possible to combine a gel based on polynucleotides with a biocompatible crosslinking agent. According to some embodiments, the process for preparing the composition of the disclosure comprises dissolving the polynucleotides in water, and then adding an alcohol, typically ethyl alcohol, to bring to complete dissolution without substantial formation of lumps. In a later step of the process, the crosslinking reagent is added to the to the solution containing the polynucleotides (PN) under stirring, obtaining a turbid solution containing least partially crosslinked PN. Typically, the resulting solution has a basic pH, for example in the range from 10 to 13.

In a later step, the pH of the crosslinked PN solution is brought to slightly acid values, for example around pH 6.2-6.6, adding a weak acid such as acetic acid. In these conditions, a fluid gel is formed to which water is subsequently added until a gel with homogeneous and consistent mass is obtained. In a later step, an alcohol is added to the gel under stirring and after decantation, a precipitate is added which conveniently in a later step can be resuspended in alcohol, typically ethyl alcohol, under stirring and precipitated and subsequently dried.

The powder obtained based on crosslinked PN can be rehydrated by adding a solvent, typically water and preferably a buffer, such as phosphate/chloride to obtain a composition in gel form.

The resulting gel may be sterilized also after packaging, for example by heating in a syringe vial.

### Definitions

In the present document, the term "carrier" refers to an excipient, carrier, diluent or adjuvant which may be present in the composition of the invention. Any carrier and/or excipient suitable for the desired form of preparation for administration is envisaged in the uses described herein.

The term "cosmetically acceptable" as used herein means that the composition or components thereof are suitable for cosmetic use and when applied, do not cause toxicity, allergic response, redness, incompatibility, instability and similar undesired reactions.

The term "physiologically acceptable" means that the substance, when applied or administered to the human body, does not cause toxicity, allergic response, redness, incompatibility, instability and similar undesired reactions.

Within this scope, the term "nucleic acid polymer chain or DNA", "nucleic acid strand or DNA" have substantially the same meaning.

In general, the term "crosslinked" refers to intermolecular bonds that bind molecules or individual polymer chains or monomeric moieties thereof in a more stable structure. Typically, a polynucleotide comprises at least one crosslinked intermolecular bond which binds at least one individual polynucleotide (PN) or a moiety thereof with another polynucleotide or a moiety thereof.

Within the scope of invention, the term "monophasic gel" means a network of crosslinked viscoelastic gel comprising less than 20%, less than 10%, less than 5% by weight of gel particles.

The term biocompatible means a product or products that are physiologically acceptable and that when injected do not cause significant adverse reactions or anyway if they occur, they are of local type and are among the reactions and discomfort commonly encountered upon injection or implantation of products of known use.

The following examples are provided primarily to illustrate the present invention and are not intended to limit the scope of protection as is clear from the appended claims.

### EXAMPLE 1

| Filler | composition |
|---|---|
| Cross-linked polynucleotides | 25 mg |
| Sodium choride | 8 mg |
| Sodium phosphate monobasic dihydrate | 0.30 mg |
| Sodium phosphate monobasic dodecahydrate | 1.5 mg |
| Water | as needed to 1 ml |

| CONTROLS | SPECIFICATIONS | 90 mg/g BDDE |
|---|---|---|
| Appearance | Compact gel | Compact gel |
| Colour | Colourless | Colourless |
| pH | 6.5 - 7.5 | 7.2 |
| Extractable volume | 1.0 - 1.1 ml | ----- |
| Osmolarity | 270-330 mOs/kg | 319 |
| Residual Bdde | < 10 ppm | conforming |
| proteins | < 0.5 % | conforming |
| Residual ethanol | < 500ppm | conforming |
| Polynucleotides UV identification | Maximum absorption at 260 ± 2 nm | conforming |
| Polynucleotides dosage | 21-29 mg/ml | 28 mg/ml |
| Sterility | Sterile | Sterile |
| Endotoxins | < 87.5 EU/ml | conforming |

### EXAMPLE 2

Process for obtaining crosslinked polynucleotides.
1A. In a beaker containing 50 ml of 1% NaOH, bring 3 g of PN to dissolution.
1B. Slowly add 45 ml of alcohol C and stir until a homogeneous solution without lumps in suspension is obtained.
1C. Add 90 mcl BDDE/gr of PN, let stir for at least 1 hour at room temperature.
2A. Adjust the pH of the sample to pH 6.4 with glacial acetic acid diluted 1:5.
2B. Add 50 ml of water and stir until a homogeneous mass is obtained.
2C. Slowly add 250 ml of alcohol, let stir for at least 30 minutes. Decant.
2D. Discard the supernatant and add 100 ml of ethyl alcohol and let stir for at least 2 hours.
3A. Decant and discard the supernatant, transfer the precipitate in a calibrated flask with emery cone and dry in vacuum in bain-marie. 50 °C for at least 1 hour.
3B. Take about 500 mg of the resulting powder and place it in a calibrated crucible; put the sample in an oven at 105 °C until the weight is constant (about 3-4 hours). Retrieve the crucible, let cool in a desiccator and weigh again. Proceed with the calculation of the loss in weight of the sample.
3C. Calculate the amount of phosphate buffer needed to rehydrate the powder to the theoretical concentration of 25 mg/ml.
3D. After adding the buffer to the powder, rehydrate in bain-marie at 50 °C for at least 8 hours.

Distribute the gel in a syringe vial and autoclave for 15 minutes at 121 °C.

### EXAMPLE 3

Experimental biocompatibility tests

| | | |
|---|---|---|
| cytotoxicity | non cytotoxic | conforming |
| Intra-cutaneous reactivity | satisfactory | conforming |
| Delayed hypersensitivity | Non sensitizing | conforming |
| Mutagenesis | non mutagenic | conforming |
| Subcutaneous implantation | no degenerative phenomena | conforming |

## Claims

1. Cosmetic or aesthetic use of an injectable composition based on a nucleic acid polymer chain moiety as dermal filler and cell regenerative or to increase the volume of a tissue, wherein the nucleic acid polymer chain moiety is at least partially crosslinked with a biocompatible crosslinking agent, whereby methods for treatment of the human or animal body by surgery or therapy are excluded.

2. Use according to claim 1, **characterized in that** said nucleic acid polymer chain moiety comprises deoxyribonucleotides linked by a phosphodiester bond.

3. Use according to claim 1 or 2, **characterized in that** said biocompatible crosslinking reagent is selected from crosslinking agents based on dialdehydes and disulfides, PEG-based crosslinking agents and mixtures thereof.

4. Use according to any one of claims 1-3, **characterized in that** said crosslinking reagent is selected from polyethylene glycol diglycil ether, polyethylene glycol epoxide, divinyl sulfone (DVS), pentaerythritol tetraglycidyl ether (PETGE), 1,4-butanediol diglycidyl ether (BDDE), 1,2-bis(2,3-epoxypropoxy)ethylene (EDGE), 1,2,7,8-diepoxyoctane (DEO), phenylene-bis-(ethyl)-carbodiimide, 1,6-hexane-ethylene bis-(ethylcarbodiimide), adipic dihydrazide (ADH), bis(sulfosuccinimidyl)suberate (BS), hexamethylenediamine (HMDA), 1-(2,3-epoxypropyl)-2,3-epoxycyclohexane, and/or mixtures thereof.

5. Use according to any one of claims 1-4, wherein said composition further comprises a physiologically acceptable carrier.

6. Use according to claim 5, wherein said physiologically acceptable carrier is selected from water, saline with phosphate buffer, glycerol, carboxymethylcellulose and mixtures thereof.

7. Use according to any one of claims 1-6, wherein said injectable composition is in the form of an injectable monophasic gel.

8. Use according to any one of claims 1-7, wherein said composition further comprises one or more substances active in regenerating the skin.

9. Use according to any one of claims 1-8 for stretching wrinkles, furrows or skin folds.

10. An injectable composition based on polynucleotides comprising a nucleic acid polymer chain moiety, wherein the nucleic acid polymer chain moiety is at least partially crosslinked with a biocompatible crosslinking agent for use as filler and cell regenerative in methods for treatment of the human or animal body by therapy or surgery, wherein the treatments are treatment of anatomical malformations with an impact on the aesthetic appearance of a person, treatment of urinary incontinence, vesicourethral reflux, gastroesophageal reflux or lipoatrophy, and in reconstructive surgery, maxillofacial surgery especially in blepharoplasty or rhinoplasty.

11. An injectable composition for use according to claim 10, wherein said biocompatible crosslinking reagent is selected from crosslinking agents based on dialdehydes and disulfides, PEG-based crosslinking agents and mixtures thereof.

12. An injectable composition for use according to claims 11, wherein said crosslinking reagent is selected from polyethylene glycol diglycil ether, polyethylene glycol epoxide, divinyl sulfone (DVS), pentaerythritol tetraglycidyl ether (PETGE), 1,4-butanediol diglycidyl ether (BDDE), 1,2-bis(2,3-epoxypropoxy)ethylene (EDGDE), 1,2,7,8-diepoxyoctane (DEO), phenylene-bis-(ethyl)-carbodiimide; 1,6-hexane-ethylene bis-(ethylcarbodiimide), adipic dihydrazide (ADH), bis(sulfosuccinimidyl)suberate (BS), hexamethylenediamine (HMDA), 1-(2,3-epoxypropyl)-2,3-epoxycyclohexane, and/or mixtures thereof.

13. An injectable composition for use according to any one of claims 10-12, further including a physiologically acceptable carrier selected from water, saline with phosphate buffer, glycerol, carboxymethylcellulose and mixtures thereof.

14. An injectable composition for use according to any one of claims 10-13 in the form of a monophasic gel.

## Patentansprüche

1. Kosmetische oder ästhetische Verwendung einer injizierbaren Zusammensetzung basierend auf einem Nukleinsäure-Polymerkettenanteil als Dermalfüller und Zellerneuerung oder zur Steigerung des Volumens eines Gewebes, wobei der Nukleinsäure-Polymerkettenanteil zumindest teilweise mit einem biokompatiblen Vernetzungsmittel vernetzt ist, wobei jedoch Verfahren zur Behandlung des menschlichen oder tierischen Körpers durch Chirurgie oder Therapie ausgeschlossen sind.

2. Verwendung gemäß Anspruch 1, **dadurch gekennzeichnet, dass** der Nukleinsäure-Polymerkettenanteil Desoxyribonukleotide aufweist, die durch eine Phosphodiesterbindung verbunden sind.

3. Verwendung gemäß Anspruch 1 oder 2, **dadurch gekennzeichnet, dass** das biokompatible Vernetzungsreagens ausgewählt ist aus Vernetzungsmitteln, die auf Dialdehyden und Disulfiden, PEG-basierenden Vernetzungsmitteln und Mischungen davon basieren.

4. Verwendung gemäß einem der Ansprüche 1 bis 3, **dadurch gekennzeichnet, dass** das Vernetzungsreagens ausgewählt ist aus Polyethylen-Glykol-Diglycil-Ether, Polyethylen-Glykol-Epoxid, Divinyl-Sulfon (DVS), Pentaerythrittetraglycidylether (PETGE), 1,4-Butandiol-Diglycil-Ether (BDDE), 1,2-bis(2,3-Epoxypropoxy)-Ethylen (EDGE), 1,2,7,8-Diepoxyoktan (DEO), Phenylen-bis-(Ethyl)-Carbodiimid, 1,6-Hexan-Ethylen-bis-(Ethylcarbodiimid), Adipinsäuredihydrazid (ADH), Bis(sulfosuccinimidyl)-Suberat (BS), Hexamethylendiamin (HMDA), 1-(2,3-Epoxypropyl)-2,3-Epoxyzyklohexan und / oder Mischungen davon.

5. Verwendung gemäß einem der Ansprüche 1 bis 4, wobei die Zusammensetzung des Weiteren einen physiologisch akzeptablen Trägerstoff aufweist.

6. Verwendung gemäß Anspruch 5, wobei der physiologisch akzeptable Trägerstoff ausgewählt ist aus Wasser, Kochsalzlösung mit Phosphatpuffer, Glyzerin, Carboxymethylcellulose und Mischungen davon.

7. Verwendung gemäß einem der Ansprüche 1 bis 6, wobei die injizierbare Zusammensetzung in Form eines injizierbaren monophasischen Gels vorliegt.

8. Verwendung gemäß einem der Ansprüche 1 bis 7, wobei die Zusammensetzung des Weiteren eine oder mehrere Substanzen aufweist, die bei der Hautregeneration aktiv sind.

9. Verwendung gemäß einem der Ansprüche 1 bis 8 zur Linderung von Fältchen, Runzeln oder Hautfalten.

10. Injizierbare Zusammensetzung basierend auf Polynukleotiden, die einen Nukleinsäure-Polymerkettenanteil aufweisen, wobei der Nukleinsäure-Polymerkettenanteil zur Verwendung als Füllstoff und Zellerneuerer bei Verfahren zur Behandlung des menschlichen oder tierischen Körpers durch Therapie oder Chirurgie zumindest teilweise mit einem biokompatiblen Vernetzungsmittel vernetzt ist, wobei die Behandlungen sind: die Behandlung von anatomischen Fehlbildungen mit Auswirkungen auf das ästhetische Erscheinungsbild einer Person, Behandlung von Harninkontinenz, vesikoureteralem Reflux, gastroösophagealem Reflux oder Lipoatrophie und Behandlung in der rekonstruktiven Chirurgie und der Gesichtschirurgie, insbesondere in der Blepharoplastik oder der Rhinoplastik.

11. Injizierbare Zusammensetzung zur Verwendung gemäß Anspruch 10, wobei das biokompatible Vernetzungsreagens ausgewählt ist aus Vernetzungsmitteln, die auf Dialdehyden und Disulfiden, PEG-basierenden Vernetzungsmitteln und Mischungen davon basieren.

12. Injizierbare Zusammensetzung zur Verwendung gemäß Anspruch 11, wobei das Vernetzungsreagens ausgewählt ist aus Polyethylen-Glykol-Diglycil-Ether, Polyethylen-Glykol-Epoxid, Divinyl-Sulfon (DVS), Pentaerythrittetraglycidylether (PETGE), 1,4-Butandiol-Diglycil-Ether (BDDE), 1,2-bis(2,3-Epoxypropoxy)-Ethylen (EDGE), 1,2,7,8-Diepoxyoktan (DEO), Phenylen-bis-(Ethyl)-Carbodiimid, 1,6-Hexan-Ethylen-bis-(Ethylcarbodiimid), Adipinsäuredihydrazid (ADH), Bis(sulfosuccinimidyl)-Suberat (BS), Hexamethylendiamin (HMDA), 1-(2,3-Epoxypropyl)-2,3-Epoxyzyklohexan und / oder Mischungen davon.

13. Injizierbare Zusammensetzung zur Verwendung gemäß einem der Ansprüche 10 bis 12, weiter aufweisend einen physiologisch akzeptablen Trägerstoff, ausgewählt aus Wasser, Kochsalzlösung mit Phosphatpuffer, Glyzerin, Carboxymethylcellulose und Mischungen davon.

14. Injizierbare Zusammensetzung zur Verwendung gemäß einem der Ansprüche 10 bis 13 in Form eines monophasischen Gels.

## Revendications

1. Utilisation cosmétique ou esthétique d'une composition injectable à base d'une fraction de chaîne polymère d'acide nucléique en tant que charge dermique et régénérateur cellulaire ou pour augmenter le volume d'un tissu, dans laquelle la fraction de chaîne polymère d'acide nucléique est au moins partiellement réticulée avec un agent de réticulation biocompatible, étant ainsi exclus des procédés de traitement du corps humain ou animal par chirurgie ou thérapie.

2. Utilisation selon la revendication 1, **caractérisée en ce que** ladite fraction de chaîne polymère d'acide nucléique comprend des désoxyribonucléotides liés par une liaison phosphodiester.

3. Utilisation selon la revendication 1 ou la revendication 2, **caractérisée en ce que** ledit réactif de réticulation biocompatible est sélectionné parmi des agents de réticulation à base de dialdéhydes et de disulfures, des agents de réticulation à base de PEG et des mélanges de ceux-ci.

4. Utilisation selon l'une quelconque des revendications 1 à 3, **caractérisée en ce que** ledit réactif de réticulation est sélectionné parmi le polyéthylène glycol diglycil éther, le polyéthylène glycol époxyde, le divinyl sulfone (DVS), le pentaérythritol tétraglycidyl éther (PETGE), le 1,4-butanediol diglycidyl éther (BDDE), le 1,2-bis(2,3-époxypropoxy)éthylène (EDGE), le 1,2,7,8-diépoxyoctane (DEO), le phénylène-bis-(éthyl)-carbodiimide, le 1,6-hexane-éthylène bis-(éthylcarbodiimide), le dihydrazide adipique (ADH), le bis(sulfosuccinimidyl)suberate (BS), l'hexaméthylènediamine (HMDA), le 1-(2,3-époxypropyl)-2,3-époxycyclohexane et/ou des mélanges de ceux-ci.

5. Utilisation selon l'une quelconque des revendications 1 à 4, dans laquelle ladite composition comprend en outre un véhicule physiologiquement acceptable.

6. Utilisation selon la revendication 5, dans laquelle ledit véhicule physiologiquement acceptable est sélectionné parmi l'eau, une solution saline avec un tampon phosphate, le glycérol, la carboxyméthylcellulose et des mélanges de ceux-ci.

7. Utilisation selon l'une quelconque des revendications 1 à 6, dans laquelle ladite composition injectable est sous la forme d'un gel monophasique injectable.

8. Utilisation selon l'une quelconque des revendications 1 à 7, dans laquelle ladite composition comprend en outre une ou plusieurs substances actives dans la régénération de la peau.

9. Utilisation selon l'une quelconque des revendications 1 à 8 pour étirer les rides, les sillons ou les plis cutanés.

10. Une composition injectable à base de polynucléotides comprenant une fraction de chaîne polymère d'acide nucléique, dans laquelle la fraction de chaîne polymère d'acide nucléique est au moins partiellement réticulée avec un agent de réticulation biocompatible pour une utilisation en tant que charge et régénérateur cellulaire dans des procédés de traitement du corps humain ou animal par thérapie ou chirurgie, dans laquelle les traitements sont le traitement de malformations anatomiques avec un impact sur l'aspect esthétique d'une personne, le traitement de l'incontinence urinaire, du reflux vésico-urétral, du reflux gastro-oesophagien ou de la lipoatrophie, et dans la chirurgie reconstructive, la chirurgie maxillo-faciale, en particulier dans la blépharoplastie ou la rhinoplastie.

11. Une composition injectable pour une utilisation selon la revendication 10, dans laquelle ledit réactif de réticulation biocompatible est sélectionné parmi des agents de réticulation à base de dialdéhydes et de disulfures, des agents de réticulation à base de PEG et des mélanges de ceux-ci.

12. Une composition injectable pour une utilisation selon la revendication 11, dans laquelle ledit réactif de réticulation est sélectionné parmi le polyéthylène glycol diglycil éther, le polyéthylène glycol époxyde, le divinyl sulfone (DVS), le pentaérythritol tétraglycidyl éther (PETGE), le 1,4-butanediol diglycidyl éther (BDDE), le 1,2-bis(2,3-époxypropoxy)éthylène (EDGE), le 1,2,7,8-diépoxyoctane (DEO), le phénylène-bis-(éthyl)-carbodiimide, le 1,6-hexane-éthylène bis-(éthylcarbodiimide), le dihydrazide adipique (ADH), le bis(sulfosuccinimidyl)suberate (BS), l'hexaméthylènediamine (HMDA), le 1-(2,3-époxypropyl)-2,3-époxycyclohexane et/ou des mélanges de ceux-ci.

13. Une composition injectable pour une utilisation selon l'une quelconque des revendications 10 à 12, incluant en outre un véhicule physiologiquement acceptable sélectionné parmi l'eau, une solution saline avec un tampon phosphate, le glycérol, la carboxyméthylcellulose et des mélanges de ceux-ci.

14. Une composition injectable pour une utilisation selon l'une quelconque des revendications 10 à 13 sous la forme d'un gel monophasique.
